(19) Europäisches Patentamt / European Patent Office / Office européen des brevets

(11) **EP 2 558 971 B1**

(12) **EUROPEAN PATENT SPECIFICATION**

(45) Date of publication and mention
of the grant of the patent:
**17.02.2021 Bulletin 2021/07**

(21) Application number: **11769387.9**

(22) Date of filing: **11.04.2011**

(51) Int Cl.:
*G16B 40/00* (2019.01)　　　　*G16B 25/20* (2019.01)
*C12Q 1/6851* (2018.01)

(86) International application number:
**PCT/US2011/031988**

(87) International publication number:
**WO 2011/130184 (20.10.2011 Gazette 2011/42)**

(54) **SYSTEMS AND METHODS FOR MODEL-BASED qPCR**

SYSTEME UND VERFAHREN FÜR QPCR AUF MODELLBASIS

SYSTÈMES ET MÉTHODES POUR LA QPCR BASÉE SUR UN MODÈLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.04.2010 US 322895 P**

(43) Date of publication of application:
**20.02.2013 Bulletin 2013/08**

(73) Proprietor: **Life Technologies Corporation
Carlsbad, CA 92008 (US)**

(72) Inventor: **GEORGE, Wallace R.
Oakland
CA 94611 (US)**

(74) Representative: **Hoffmann Eitle
Patent- und Rechtsanwälte PartmbB
Arabellastraße 30
81925 München (DE)**

(56) References cited:
　JP-A- 2005 504 543　　JP-A- 2007 128 483
　KR-A- 20000 016 326　　US-A1- 2007 260 440

• **RUTLEDGE ROBERT G ET AL: "A kinetic-based sigmoidal model for the polymerase chain reaction and its application to high-capacity absolute quantitative real-time PCR", BMC BIOTECHNOLOGY, BIOMED CENTRAL LTD. LONDON, GB, vol. 8, no. 1, 8 May 2008 (2008-05-08), page 47, XP021035719, ISSN: 1472-6750**
• **C. RITZ ET AL: "qpcR: an R package for sigmoidal model selection in quantitative real-time polymerase chain reaction analysis", BIOINFORMATICS, vol. 24, no. 13, 1 July 2008 (2008-07-01), pages 1549-1551, XP055042712, ISSN: 1367-4803, DOI: 10.1093/bioinformatics/btn227**
• **Anonymous: "Crt, a relative threshold method for qPCR data analysis on the QuantStudio 12K Flex system with OpenArray technology", , 1 March 2016 (2016-03-01), XP055719954, Retrieved from the Internet: URL:https://assets.thermofisher.com/TFS-Assets/LSG/brochures/CO28730-Crt-Tech-note_FLR.pdf [retrieved on 2020-08-04]**

## Description

### BACKGROUND

Polymerase Chain Reaction (PCR) instrumentation has made it possible to perform reliable quantification of DNA or RNA levels in biological samples. Commercially available PCR instruments, and related data acquisition and analysis software, process qPCR assay data generated from biological samples. These systems report quantitative results by calculating a threshold cycle ($C_T$ or $C_{RT}$) value as the fractional PCR cycle number where the reporter signal rises above a threshold set manually by a human or automatically by software. The determined $C_T$ value can be used to estimate the initial quantity of DNA material.

Various approaches for the analysis of PCR amplification curves known in the art may rely, at least in part, on defining a linear exponential range for a portion of a PCR amplification curve in order to determine a cycle threshold for a sample. Approaches using modeling of a PCR amplification curve to determine a cycle threshold value for a sample may be difficult to use successfully, due to the complexity of modeling such curves. Various models for a PCR amplification curve require at least about 15 parameters or more in order to approximately model a PCR amplification curve. Furthermore, the beginning and later points in an amplification curve may be noisy and insensitive to parameter change.
US 2007/260440 A1 attempts to determine an initial quantity of nucleic acids in a sample subjected to PCR in real time. To overcome the disadvantage of having a user define a fluorescence threshold, US 2007/260440 A1 proposes to (a) provide a model of the yield of the amplification reaction as a function of the succession of amplifications, wherein the model comprises: a substantially constant stage for a first portion of the applications of the amplification reaction; and a non-constant stage for a second portion of the applications of the amplification reaction, wherein the first and second portions being united by a changeover region in which yield changes over between the constant and non-constant stages, said region having an amplification index corresponding substantially to the changeover; (b) use the yield model to express a relationship involving at least the changeover index and a parameter representative of the initial population size in the sample of interest, and (c) determine at least the changeover index by comparison with the experimental measurements; and, in a subsequent or immediately following step (d) deduce therefrom the initial population size in the sample of interest.

RUTLEDGE ROBERT G ET AL, "A kinetic-based sigmoidal model for the polymerase chain reaction and its application to high-capacity absolute quantitative real-time PCR", BMC BIOTECHNOLOGY, BIOMED CENTRAL LTD. LONDON, GB, (20080508), vol. 8, no. 1, ISSN 1472-6750, page 47, XP021035719 recognizes that amplification rate is linearly correlated to amplicon quantity led to the derivation of two sigmoid functions that allow target quantification via linear regression analysis. In addition to circumventing quantitative errors produced by plateau distortions, the amplification efficiency within individual amplification reactions may be determined. Absolute quantification is accomplished by first converting individual fluorescence readings into target quantity expressed in fluorescence units, followed by conversion into the number of target molecules via optical calibration. Founded upon expressing reaction fluorescence in relation to amplicon DNA mass, the article proposes to implement optical calibration using lambda gDNA as a universal quantitative standard. This eliminates the need to prepare target-specific quantitative standards, it relegates establishment of quantitative scale to a single, highly defined entity.

Further, C. RITZ ET AL, "qpcR: an R package for sigmoidal model selection in quantitative real-time polymerase chain reaction analysis", BIOINFORMATICS, (20080701), vol. 24, no. 13, doi:10.1093/bioinformatics/btn227, ISSN 1367-4803, pages 1549 - 1551, XP055042712 proposes a statistical model selection process for sigmoidal fitting of real-time PCR data which includes a five-parameter sigmoidal model with an additional parameter for asymmetric data in the context of qPCR analysis.

### SUMMARY

In one embodiment, a computer-implemented method for determining a cycle threshold for a PCR amplification curve is defined by claim 1. Preferred embodiments are defined by claims 2 and 3. In a further embodiment, a computer-readable medium is defined by claim 4. In yet a further embodiment, a system for determining a cycle threshold for a PCR amplification curve is defined by claim 5.

### BRIEF DESCRIPTION OF THE DRAWINGS

FIG. 1 is a flow chart that depicts various embodiments of systems and methods for the analysis of qPCR sample amplification curve data.
FIG. 2 is a block diagram of a PCR instrument that may be used in the PCR amplification of samples.

FIG. 3 is a block diagram that illustrates components of an exemplary computer system that may be utilized in the control and interface of PCR instrumentation.

FIG. 4 is a graphical representation of a modeled efficiency and associated modeled scaled amplification curve along with an actual amplification curve according to various embodiments of systems and methods of model-based qPCR.

FIG. 5 is a graphical representation of varying an $X_{shift}$ parameter on various embodiments of systems and methods of model-based qPCR.

FIG. 6 is a graphical representation of varying an $\alpha_{bend}$ parameter on various embodiments of systems and methods of model-based qPCR.

FIG. 7 is a graphical representation of varying an $x_0'$ relative shift parameter on various embodiments of systems and methods of model-based qPCR.

FIG. 8 is a graphical representation of varying the $\alpha_{amp}$ and rising baseline parameter on various embodiments of systems and methods of model-based qPCR.

FIG. 9 is a tabular summary of various embodiments of tests performed for the detection of non-amplification samples.

FIG. 10 is a graphical representation of a noise-normalized amplification curve amplitudes versus adjusted $X_{shift}$ values for various amplified and non-amplified curves/wells according to various embodiments.

## DETAILED DESCRIPTION

The present teachings relate to embodiments of methods and systems for the analysis of PCR amplification curves for a plurality of biological samples. The invention is set out in the appended set of claims. Any references in the following description to embodiments, objects, aspects and/or examples which are not covered by the appended claims are considered as not being part of the present invention.

As described in the MIQE Guidelines, fractional cycle values computed via various alternative methods (with associated nomenclature) appear in the literature. (Bustin SA, Benes V, Garson JA, Hellemans J, Huggett J, Kubista M, Mueller R, Nolan T, Pfaffl MW, Shipley GL, Vandesompele J, Wittwer, CT: The MIQE guidelines: minimum information for publication of quantitative real-time PCR experiments. Clin Chem 2009, 55:611-622.) Following MIQE recommendations, this document will use $C_Q$ when referring generically to fractional qPCR cycle values. However, when we specifically need to distinguish between Baseline Threshold ($C_T$) and Relative Threshold ($C_{RT}$) methods, $C_T$ and $C_{RT}$ will be used respectively.

According to various embodiments of systems and methods of the present teachings for a model-based determination of a cycle threshold use as a basis a model of the reaction efficiency, from which a model amplification curve may be created. For various embodiments, a constrained nonlinear optimization of an amplification model may be performed to determine a set of best-fit parameters for a sample amplification curve. Various embodiments may then use a set of best-fit parameters to create a modeled efficiency curve and a modeled amplification curve, from which a cycle threshold may be determined. According to various embodiments, a portion of the amplification curve may be chosen and utilized to obtain the constrained nonlinear optimization fit.

For various embodiments, preprocessing the data may include clearing to remove, for example, random noise, such as spikes, smoothing to decrease systematic noise, as well as rescaling each amplification curve to a normalized scale. In various embodiments, at least one test may be performed on the data to identified non-amplified samples. According to various embodiments, a quality value may be assigned to each cycle threshold determined for each biological sample.

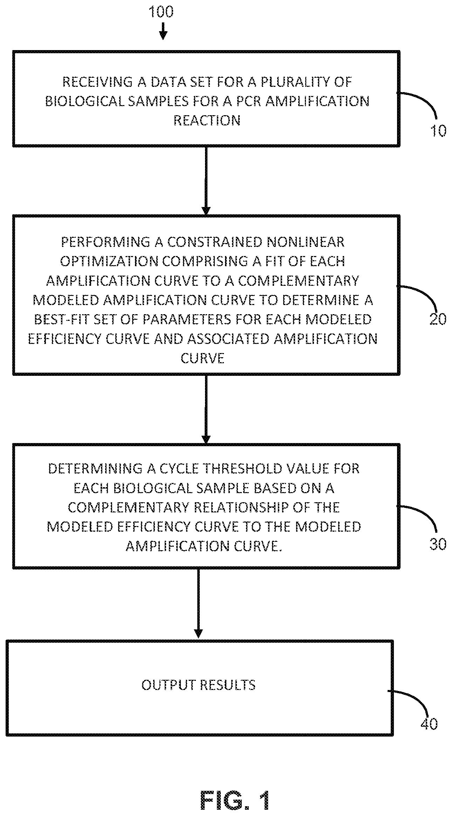

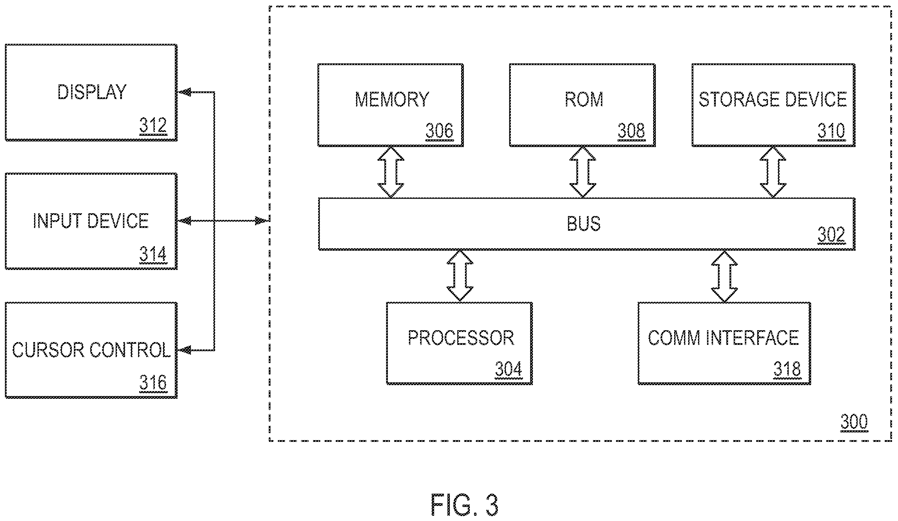

FIG. 1 depicts method 100, with steps 10-40, according to methods and systems for the analysis of PCR amplification curves of the present teachings. Method 100 may be performed by a computing system, as depicted in FIG. 3. In particular, the methods according to embodiments described herein may be performed by a processor 304 executing computer instructions stored in memory 306. For step 10 of FIG.1, a data set of PCR sample amplification curves may be received.

Various embodiments of methods and systems may utilize data sets of PCR amplification data collected over the entirety a number of amplification cycles performed for an assay. Such signals may be stored in a variety of computer readable media, and analyzed either dynamically during analysis or post analysis, as will be discussed in more details subsequently. One such type of assay used to demonstrate the features of embodiments of methods and systems for the analysis of PCR amplification data can utilize TAQMAN® fluorescent probe reagents, and may use FAM and VIC dye labels. However, one of ordinary skill in the art will recognize that a variety of assays including labeling probe reagents may be utilized to produce data that may be analyzed according to various embodiments of methods and systems of the present teachings.

The term "labeling probe" generally, according to various embodiments, refers to a molecule used in an amplification reaction, typically for quantitative or real-time PCR analysis, as well as end-point analysis. Such labeling probes may be used to monitor the amplification of the target polynucleotide. In some embodiments, oligonucleotide probes present in an amplification reaction are suitable for monitoring the amount of amplicon(s) produced as a function of time. Such oligonucleotide probes include, but are not limited to, the 5'-exonuclease assay TAQMAN® fluorescent probes described herein (see also U.S. Patent No. 5,538,848), various stem-loop molecular beacons (see e.g., U.S. Patent Nos. 6,103,476 and 5,925,517 and Tyagi and Kramer, 1996, Nature Biotechnology 14:303-308), stemless or linear beacons (see, e.g., WO 99/21881), PNA MOLECULAR BEACONS ™ (see, e.g., U.S. Patent Nos. 6,355,421 and 6,593,091), linear PNA beacons (see, e.g., Kubista et al., 2001, SPIE 4264:53-58), non-FRET probes (see, e.g., U.S. Patent No. 6,150,097), SUNRISE®/AMPLIFLUOR® probes (U.S. Patent No. 6,548,250), stem-loop and duplex Scorpion ™ probes (Solinas et al., 2001, Nucleic Acids Research 29:E96 and U.S. Patent No. 6,589,743), bulge loop probes (U.S. Patent No. 6,590,091), pseudo knot probes (U.S. Patent No. 6,589,250), cyclicons (U.S. Patent No. 6,383,752), MGB ECLIPSE™ probe (Epoch Biosciences), hairpin probes (U.S. Patent No. 6,596,490), peptide nucleic acid (PNA) light-up probes, self-assembled nanoparticle probes, and ferrocene-modified probes described, for example, in U.S. Patent No. 6,485,901; Mhlanga et al., 2001, Methods 25:463-471; Whitcombe et al., 1999, Nature Biotechnology. 17:804-807; Isacsson et al., 2000, Molecular Cell Probes. 14:321-328; Svanvik et al., 2000, Anal Biochem. 281:26-35; Wolffs et al., 2001, Biotechniques 766:769-771; Tsourkas et al., 2002, Nucleic Acids Research. 30:4208-4215; Riccelli et al., 2002, Nucleic Acids Research 30:4088-4093; Zhang et al., 2002 Shanghai. 34:329-332; Maxwell et al., 2002, J. Am. Chem. Soc. 124:9606-9612; Broude et al., 2002, Trends Biotechnol. 20:249-56; Huang et al., 2002, Chem Res. Toxicol. 15:118-126; and Yu et al., 2001, J. Am. Chem. Soc 14:11155-11161.Labeling probes can also comprise black hole quenchers (Biosearch), Iowa Black (IDT), QSY quencher (Molecular Probes), and Dabsyl and Dabcel sulfonate/carboxylate Quenchers (Epoch). Labeling probes can also comprise two probes, wherein for example a fluorophore is on one probe, and a quencher on the other, wherein hybridization of the two probes together on a target quenches the signal, or wherein hybridization on target alters the signal signature via a change in fluorescence. Labeling probes can also comprise sulfonate derivatives of fluorescenin dyes with a sulfonic acid group instead of the carboxylate group, phosphoramidite forms of fluorescein, phosphoramidite forms of CY 5 (available for example from Amersham).

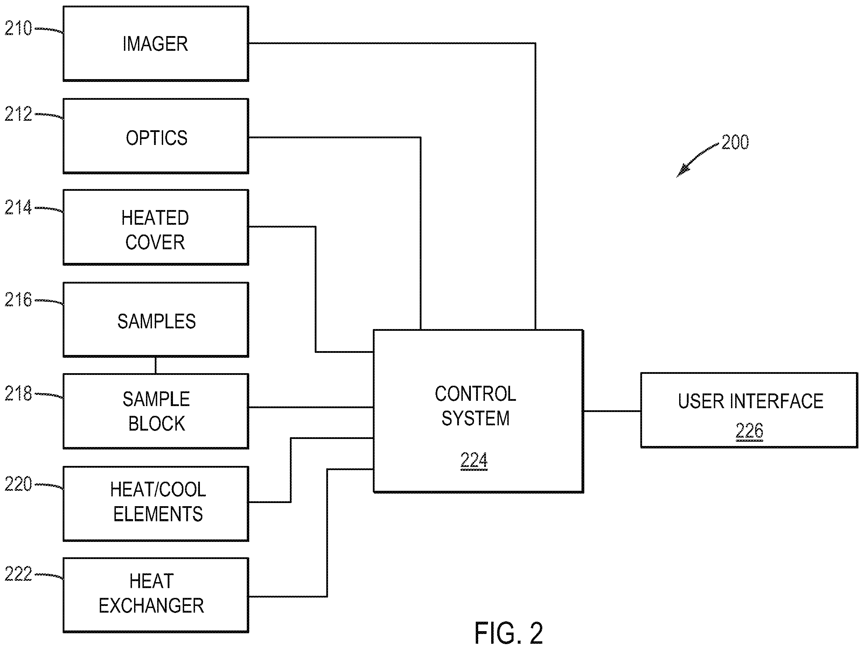

Various embodiments of methods and systems for the analysis of PCR amplification curves according to the present teachings may utilize various embodiments of a thermal cycler instrument as depicted in the block diagrams shown in FIG. 2. As shown in FIG. 2, a thermal cycling instrument may include a heated cover 214 that is placed over a plurality of samples 216 contained in a sample support device. In various embodiments, a sample support device may be a glass or plastic slide with a plurality of sample regions, which sample regions have a cover between the sample regions and heated cover 214. Some examples of a sample support device may include, but are not limited by, a multi-well plate, such as a standard microtiter 96-well, a 384-well plate, or a microcard, or a substantially planar support, such as a glass or plastic slide. The sample regions in various embodiments of a sample support device may include depressions, indentations, ridges, and combinations thereof, patterned in regular or irregular arrays formed on the surface of the substrate. In various embodiments of a thermal cycler instrument, include a sample block 218, elements for heating and cooling 220, and a heat exchanger 222.

In FIG. 2, various embodiments of a thermal cycling system 200 provide a detection system for the real-time acquisition of signals for each sample in a plurality of biological samples, over the entirety the number of amplification cycles performed for an assay. A detection system may have an illumination source that emits electromagnetic energy, and a detector or imager 210, for receiving electromagnetic energy from samples 216 in sample support device. A control system 224 may be used to control the functions of the detection, heated cover, and thermal block assembly. The control system may be accessible to an end user through user interface 226 of thermal cycler instrument 200. A computer system 300, as depicted in FIG. 3 may serve as to provide the control the function of a thermal cycler instrument, as well as the user interface function. Additionally, computer system 300 may provide data processing, display and report preparation functions. All such instrument control functions may be dedicated locally to the thermal cycler instrument, or computer system 300 may provide remote control of part or all of the control, analysis, and reporting functions, as will be discussed in more detail subsequently.

Those skilled in the art will recognize that the operations of the various embodiments may be implemented using hardware, software, firmware, or combinations thereof, as appropriate. For example, some processes can be carried out using processors or other digital circuitry under the control of software, firmware, or hard-wired logic. (The term "logic" herein refers to fixed hardware, programmable logic and/or an appropriate combination thereof, as would be recognized by one skilled in the art to carry out the recited functions.) Software and firmware can be stored on computer-readable media. Some other processes can be implemented using analog circuitry, as is well known to one of ordinary skill in the art. Additionally, memory or other storage, as well as communication components, may be employed in embodiments of the invention.

FIG. 3 is a block diagram that illustrates a computer system 300 that may be employed to carry out processing functionality, according to various embodiments, upon which embodiments of a thermal cycler system 200 of FIG. 2. Computing system 300 can include one or more processors, such as a processor 304. Processor 304 can be implemented using a general or special purpose processing engine such as, for example, a microprocessor, controller or other control logic. In this example, processor 304 is connected to a bus 302 or other communication medium.

Further, it should be appreciated that a computing system 300 of FIG. 3 may be embodied in any of a number of forms, such as a rack-mounted computer, mainframe, supercomputer, server, client, a desktop computer, a laptop computer, a tablet computer, hand-held computing device (e.g., PDA, cell phone, smart phone, palmtop, etc.), cluster grid, netbook, embedded systems, or any other type of special or general purpose computing device as may be desirable or appropriate for a given application or environment. Additionally, a computing system 300 can include a conventional network system including a client/server environment and one or more database servers, or integration with LIS/LIMS infrastructure. A number of conventional network systems, including a local area network (LAN) or a wide area network (WAN), and including wireless and/or wired components, are known in the art. Additionally, client/server environments, database servers, and networks are well documented in the art.

Computing system 300 may include bus 302 or other communication mechanism for communicating information, and processor 304 coupled with bus 302 for processing information.

Computing system 300 also includes a memory 306, which can be a random access memory (RAM) or other dynamic memory, coupled to bus 302 for storing instructions to be executed by processor 304. Memory 306 also may be used for storing temporary variables or other intermediate information during execution of instructions to be executed by processor 304. Computing system 300 further includes a read only memory (ROM) 308 or other static storage device coupled to bus 302 for storing static information and instructions for processor 304.

Computing system 300 may also include a storage device 310, such as a magnetic disk, optical disk, or solid state drive (SSD) is provided and coupled to bus 302 for storing information and instructions. Storage device 310 may include a media drive and a removable storage interface. A media drive may include a drive or other mechanism to support fixed or removable storage media, such as a hard disk drive, a floppy disk drive, a magnetic tape drive, an optical disk drive, a CD or DVD drive (R or RW), flash drive, or other removable or fixed media drive. As these examples illustrate, the storage media may include a computer-readable storage medium having stored therein particular computer software, instructions, or data.

In alternative embodiments, storage device 310 may include other similar instrumentalities for allowing computer programs or other instructions or data to be loaded into computing system 300. Such instrumentalities may include, for example, a removable storage unit and an interface, such as a program cartridge and cartridge interface, a removable memory (for example, a flash memory or other removable memory module) and memory slot, and other removable storage units and interfaces that allow software and data to be transferred from the storage device 310 to computing system 300.

Computing system 300 can also include a communications interface 318. Communications interface 318 can be used to allow software and data to be transferred between computing system 300 and external devices. Examples of communications interface 318 can include a modem, a network interface (such as an Ethernet or other NIC card), a communications port (such as for example, a USB port, a RS-232C serial port), a PCMCIA slot and card, Bluetooth, etc. Software and data transferred via communications interface 318 are in the form of signals which can be electronic, electromagnetic, optical or other signals capable of being received by communications interface 318. These signals may be transmitted and received by communications interface 318 via a channel such as a wireless medium, wire or cable, fiber optics, or other communications medium. Some examples of a channel include a phone line, a cellular phone link, an RF link, a network interface, a local or wide area network, and other communications channels.

Computing system 300 may be coupled via bus 302 to a display 312, such as a cathode ray tube (CRT) or liquid crystal display (LCD), for displaying information to a computer user. An input device 314, including alphanumeric and other keys, is coupled to bus 302 for communicating information and command selections to processor 304, for example. An input device may also be a display, such as an LCD display, configured with touchscreen input capabilities. Another type of user input device is cursor control 316, such as a mouse, a trackball or cursor direction keys for communicating direction information and command selections to processor 304 and for controlling cursor movement on display 312. This input device typically has two degrees of freedom in two axes, a first axis (e.g., x) and a second axis (e.g., y), that allows the device to specify positions in a plane. A computing system 300 provides data processing and provides a level of confidence for such data. Consistent with certain implementations of embodiments of the present teachings, data processing and confidence values are provided by computing system 300 in response to processor 304 executing one or more sequences of one or more instructions contained in memory 306. Such instructions may be read into memory 306 from another computer-readable medium, such as storage device 310. Execution of the sequences of instructions contained in memory 306 causes processor 304 to perform the process states described herein. Alternatively hard-wired circuitry may be used in place of or in combination with software instructions to implement embodiments of the present teachings. Thus implementations of embodiments of the present teachings are not limited to any specific combination of hardware circuitry and software.

The term "computer-readable medium" and "computer program product" as used herein generally refers to any media that is involved in providing one or more sequences or one or more instructions to processor 304 for execution. Such instructions, generally referred to as "computer program code" (which may be grouped in the form of computer programs or other groupings), when executed, enable the computing system 300 to perform features or functions of embodiments of the present invention. These and other forms of computer-readable media may take many forms, including but not limited to, non-volatile media, volatile media, and transmission media. Non-volatile media includes, for example, solid state, optical or magnetic disks, such as storage device 310. Volatile media includes dynamic memory, such as memory 306. Transmission media includes coaxial cables, copper wire, and fiber optics, including the wires that comprise bus 302.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor 304 for execution. For example, the instructions may initially be carried on magnetic disk of a remote computer. The remote computer can load the instructions into its dynamic memory and send the instructions over a telephone line using a modem. A modem local to computing system 300 can receive the data on the telephone line and use an infra-red transmitter to convert the data to an infra-red signal. An infra-red detector coupled to bus 302 can receive the data carried in the infra-red signal and place the data on bus 302. Bus 302 carries the data to memory 306, from which processor 304 retrieves and executes the instructions. The instructions received by memory 306 may optionally be stored on storage device 310 either before or after execution by processor 304.

It will be appreciated that, for clarity purposes, the above description has described embodiments of the invention with reference to different functional units and processors. However, it will be apparent that any suitable distribution of functionality between different functional units, processors or domains may be used without detracting from the invention. For example, functionality illustrated to be performed by separate processors or controllers may be performed by the same processor or controller. Hence, references to specific functional units are only to be seen as references to suitable means for providing the described functionality, rather than indicative of a strict logical or physical structure or organization.

In some embodiments, preprocessing each of a plurality of sample amplification curves may be done. Such prepossessing may be done in order remove, reduce and otherwise lessen the impact of untoward features in a PCR amplification curve that may interfere in the effective processing of the data through data analysis steps. In various embodiments of methods and systems, preprocessing the data may remove, for example, spikes, notches and noise using a variety of preprocessing methods. According to various embodiments, cleaning of a PCR amplification curve may be done using various methods for spike and notch filtering. In various embodiments, smoothing a PCR amplification curve may be done to reduce noise. For various embodiments, smoothing may be done using a windowed smoothing function, which may utilize any of a plurality of smoothing windows known in the art. According to various embodiments, rescaling of the PCR amplification curves may be done, in order to adjust a plurality of curves to a common scale. In various embodiments, sample PCR amplification curves may be scaled between a domain of 0-1.

With reference to step 20 of FIG. 1, for various methods and systems for the analysis of PCR amplification curves according to the present teachings, an analytical expression for the real-time RT-PCR efficiency may provide the basis for modeling PCR amplification curve data. For various embodiments, a three parameter analytical expression for the real-time RT-PCR efficiency may provide the basis for modeling PCR amplification curve data. In various embodiments, the 3 parameters may include a curve shift parameter, a curve bend parameter, and a curve shift adjustment parameter. An expression for the efficiency may then be used to, on a cycle by cycle basis, iteratively and recursively generate a PCR amplification curve, wherein the amplification curve so generated may have a defined baseline. According to various embodiments of the present teaching, a corresponding model for a PCR amplification curve may be created based on various embodiments of an efficiency model. This means that the PCR amplification curve is being implicitly modeled based on an explicit model of the PCR efficiency curve on a cycle by cycle basis. In this way, fewer parameter may be used to generate a model curve and a constrained optimization is possible.

For various embodiments of an amplification curve model based on an efficiency model, additional parameters defining the model may be added. In various embodiments, a 5-parameter amplification curve model may include a slope parameter for an amplification curve baseline and a relative amplification parameter. In various embodiments, a constrained non-linear optimization may be used, wherein a best-fit set of parameters for an amplification curve model may be determined for each amplification curve.

It should be recognized that embodiments are not limited to three or five parameters. For example, according to some embodiments, four or more parameters may be used to model an efficiency or amplification curve.

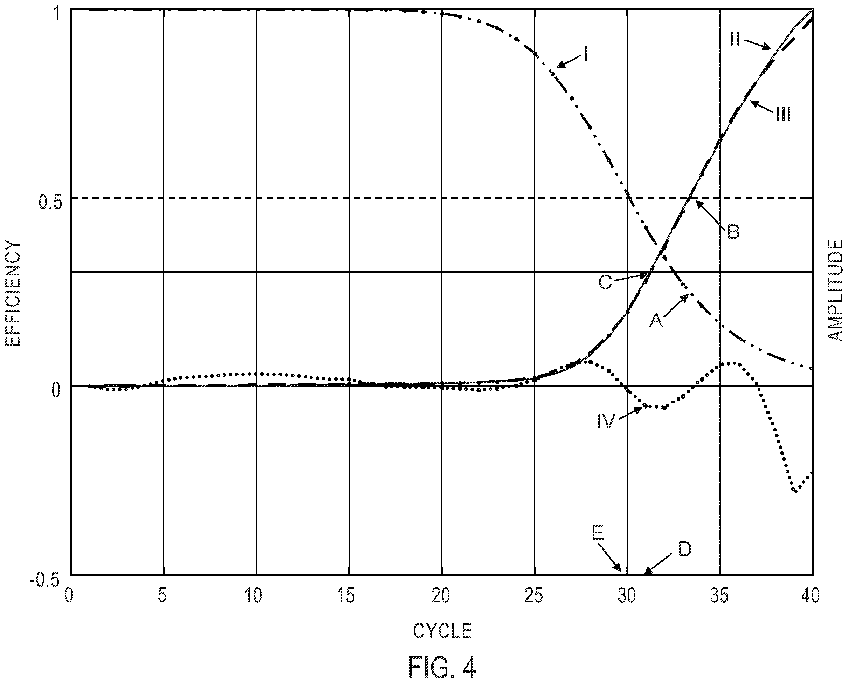

As depicted in step 20 of FIG. 1, and in reference to FIG. 4, for various embodiments of systems and methods of the present teachings, once a best-fit set of parameters for each sample amplification curve has been determined, a modeled efficiency curve and a modeled amplification curve may be created. In FIG. 4, curve I is a modeled efficiency curve, curve II is a sample amplification curve, and curve III is a modeled amplification curve. Various embodiments of methods and systems using a 5-parameter amplification curve model may provide a close fit of a modeled amplification curve to a sample amplification curve, as depicted in plot IV (which shows 10 times the difference between curve III and curve II). As a measure of fit, an expression for the residual difference between the data of a sample amplification curve and a modeled amplication curve may be generated for each of a plurality of points across the curves. For plot IV, such a measure of fit is graphically displayed, in which the residual has been multiplied by a factor of 10 in order to observe the residual across curves II and III.

The efficiency at nth discrete (fractional) cycle step $n\Delta x$ is assumed to be a smooth monotonically decreasing function, typically (but not necessarily) starting from 1 (for fractional cycle step n = 1) and trending towards 0 (for cycle step n = ∞). Here the cycle step size discretization is given by $\Delta x$. Note that, in the typical case, when $\Delta x$ is 1, then $n\Delta x$ becomes the nth cycle. In various embodiments, the efficiency is modeled in terms of a fluorescence curve shift parameter ($X_{shift}$), a fluorescence curve bend parameter ($\alpha_{bend}$), and shift adjustment parameter ($x_0$).

According to various embodiments of systems and methods of FIG. 1, step 20, a 3-parameter efficiency model may be given as:

$$e\left(n\Delta x,\, X_{shift},\, \alpha_{bend},\, x_0\right) \cong \frac{1}{\left[abs\left(1 + \left(\frac{n\Delta x - x_0}{X_{shift}}\right)^{X_{shift}^{\alpha_{bend}}}\right)\right]}$$

where:

$n\Delta x$ = the nth discrete cycle step;
$X_{shift}$ = an amplification curve shift parameter;
$\alpha_{bend}$ = an amplification curve bend parameter; and
$x_0$ = a relative shift adjustment parameter.

rearranging, the model may be written as:

$$e\left(n\Delta x,\, X_{shift},\, \alpha_{bend},\, x_0'\right) \cong \frac{1}{\left[abs\left(1 + \left(\frac{n\Delta x}{X_{shift}} - x_0'\right)^{X_{shift}^{\alpha_{bend}}}\right)\right]}$$

where: $x_0' \equiv \dfrac{x_0}{X_{shift}}$ is a normalized relative shift adjustment parameter.

It can be noted that the level of gene expression scales the rate at which the efficiency approaches zero as the number of cycles increases. In other words, when the gene expression is high, the efficiency approaches zero at a relatively small number of cycles. Conversely when the gene expression is low, the efficiency approaches zero at a relatively large number of cycles. Thus, a gene expression proxy parameter, a parameter that is strongly correlated with gene expression, may act as a scaling factor for the number of reaction cycles.

It can be recognized that when $n\Delta x = X_{shift} + x_0$, the efficiency becomes exactly 0.5. Thus, the sum ($X_{shift} + x0$) is the fractional "half-efficiency" cycle. Thus, in the equation given above for the efficiency, $X_{shift} + X_0$ plays the role of the gene expression proxy. The actual gene expression is determined using other techniques.

An important part of this efficiency equation is the $X_{shift}^{\alpha_{bend}}$ exponent in the denominator. This reflects that the exponent is a function of $X_{shift}$. In this way, the $\alpha_{bend}$ changes very little from dataset to dataset in this equation. As such, the non-linear optimization is more robust.

Upon defining

$$E\left(n\Delta x, X_{shift}, \alpha_{bend}, x_0\right) \equiv \left(1 + e\left(n\Delta x, X_{shift}, \alpha_{bend}, x_0\right)\right)^{\Delta x}$$

According to various embodiments of systems and methods of FIG. 1, step 20, a 5-parameter amplification model may be given as:

$$f\left(n\Delta x,\ \Theta_{\text{rising baseline}},\ \alpha_{amp},\ X_{shift},\ \alpha_{bend},\ x_0\right)$$

$$\equiv \Theta_{\text{rising baseline}} * (n\Delta x) + \alpha_{amp} \prod_{n'}^{n} E\left(n'\Delta x, X_{shift}, \alpha_{bend}, x_0\right)$$

where two additional parameters for the PCR amplification curve model are:

$\Theta_{\text{rising baseline}}$ = an amplification curve rising baseline slope parameter; and
$\alpha_{amp}$ = an amplification curve relative amplification parameter.

This model can be easily be extended to more than 5 parameters by for instance adding a constant term in addition to the rising baseline term leading to a 6 parameter model. Additional obvious extensions include accounting for deviations from the 5-parameter model early or late in the PCR reaction process by using additional $X_{shift}$, $\alpha_{bend}$, $X_0$ and/or $\alpha_{amp}$ parameters leading to a 9-parameter model such as:

$$f\left(n\Delta x,\ \Theta_{\text{rising baseline}},\ \alpha_{amp,1},\ X_{shift,1},\ \alpha_{bend,1}, x_{0,1},\ \alpha_{amp,2},\ X_{shift,2},\ \alpha_{bend,2}, x_{0,2}\right)$$

$$= \Theta_{\text{rising baseline}} * (n\Delta x) + \alpha_{amp,1} \prod_{n'}^{n} E\left(n'\Delta x, X_{shift,1}, \alpha_{bend,1}, x_{0,1}\right)$$

$$+ \alpha_{amp,2} \prod_{n'}^{n} E\left(n'\Delta x, X_{shift,2}, \alpha_{bend,2}, x_{0,2}\right)$$

Similar analogous extensions include a 6-parameter model (using a constant term $c_0$) such as,

$$f\left(n\Delta x, c_0, \Theta_{\text{rising baseline}},\ \alpha_{amp,1}, X_{shift,1}, \alpha_{bend}, x_0,\ \alpha_{amp,2}, X_{shift,2}\right)$$

$$\equiv c_0 + \Theta_{\text{rising baseline}} * (n\Delta x) + \alpha_{amp,1} \prod_{n'}^{n} E\left(n'\Delta x, X_{shift,1}, \alpha_{bend}, x_0\right)$$

$$+ \alpha_{amp,2} \prod_{n'}^{n} E\left(n'\Delta x, X_{shift,2}, \alpha_{bend}, x_0\right),$$

a 7-parameter model such as,

$$f\left(n\Delta x, \Theta_{rising\ baseline}, \alpha_{amp,1}, X_{shift,1}, \alpha_{bend}, x_{0,}, \alpha_{amp,2}, X_{shift,2}\right)$$

$$\equiv \Theta_{rising\ baseline} * (n\Delta x) + \alpha_{amp,1} \prod_{n'}^{n} E\left(n'\Delta x, X_{shift,1}, \alpha_{bend}, x_{0,}\right)$$

$$+ \alpha_{amp,2} \prod_{n'}^{n} E\left(n'\Delta x, X_{shift,2}, \alpha_{bend}, x_0\right),$$

an 8-parameter model such as,

$$f\left(n\Delta x, \Theta_{rising\ baseline}, \alpha_{amp,1}, X_{shift,1}, \alpha_{bend,1}, x_{0,}, \alpha_{amp,2}, X_{shift,2}, \alpha_{bend,2}\right)$$

$$= \Theta_{rising\ baseline} * (n\Delta x) + \alpha_{amp,1} \prod_{n'}^{n} E\left(n'\Delta x, X_{shift,1}, \alpha_{bend,1}, x_{0,}\right)$$

$$+ \alpha_{amp,2} \prod_{n'}^{n} E\left(n'\Delta x, X_{shift,2}, \alpha_{bend,2}, x_0\right),$$

an 10-parameter model such as,

$$f\left(n\Delta x, c_0, \Theta_{rising\ baseline}, \alpha_{amp,1}, X_{shift,1}, \alpha_{bend,1}, x_{0,1}, \alpha_{amp,2}, X_{shift,2}, \alpha_{bend,2}, x_{0,2}\right)$$

$$\equiv c_0 + \Theta_{rising\ baseline} * (n\Delta x)$$

$$+ \alpha_{amp,1} \prod_{n'}^{n} E\left(n'\Delta x, X_{shift,1}, \alpha_{bend,1}, x_{0,1}\right)$$

$$+ \alpha_{amp,2} \prod_{n'}^{n} E\left(n'\Delta x, X_{shift,2}, \alpha_{bend,2}, x_{0,2}\right),$$

and other similar combinations of the parameterizations of the model.

Models with less than 5 parameters can also be created. Upon imposing the restriction:

$$\alpha_{amp} + \Theta_{rising\ baseline} = 1,$$

we can eliminate 1 independent parameter to create a 4-parameter model such as:

$$f\left(n\Delta x, \alpha_{amp}, X_{shift}, \alpha_{bend}, x_0\right)$$

$$\equiv \left(1 - \alpha_{amp}\right) * (n\Delta x) + \alpha_{amp} \prod_{n'}^{n} E\left(n'\Delta x, X_{shift}, \alpha_{bend}, x_0\right).$$

Similarly we can create a 3 parameter model by setting $\alpha_{bend}$ to a nominal value such as 0.67, for example,

leading to:

$$f\left(n\Delta x,\ \alpha_{amp},\ X_{shift},\alpha_{bend}\ -\ 0.67, x_0\right)$$
$$= \left(1 - \alpha_{amp}\right) * (n\Delta x) + \alpha_{amp} \prod_{n'}^{n} E\left(n'\Delta x, X_{shift}, \alpha_{bend}\ =\ 0.67, x_0\right).$$

A 2 parameter model can be similarly created by setting $x_0$ to a nominal value such as 0.0, for example, leading to:

$$f\left(n\Delta x,\ \alpha_{amp}, X_{shift}, \alpha_{bend}\ =\ 0.67, x_0\ =\ 0.0\right)$$
$$\equiv \left(1 - \alpha_{amp}\right) * (n\Delta x)$$
$$+ \alpha_{amp} \prod_{n'}^{n} E\left(n'\Delta x, X_{shift}, \alpha_{bend}\ =\ 0.67, x_0\ =\ 0.0\right).$$

Thus, the modeled fluorescence curve is determined as a logarithmic integration of the effect of the efficiency at each cycle.

A non-linear constrained optimization parameter solution for the best-fit parameters in the curve amplification model above is equivalent to a non-linear baselining step. As one of ordinary skill in the art understands, the constrained optimization can be done by a routine such as Matlab's lsqnonlin which uses trust regions along with a Newton type non-linear search technique, for example.

The advantage of an amplified fluorescence curve that is implicitly defined in terms of an explicitly defined efficiency model is that it enables a better fit over a larger number of cycles using relatively few model parameters. This results in more stable constrained nonlinear parameter optimizations/inversions. Using a constrained nonlinear optimization leads to a more stable set of solutions. The more commonly-used unconstrained optimization techniques can lead to solutions that are sometimes unstable or may result in minima that are clearly not correct. The scaled nature of the parameters in embodiments of systems and methods of model-based qPCR makes it easier to set the parameter constraints required for the constrained optimization.

Once the algorithm finds the nonlinear parameters characterizing the efficiency curve, relative thresholding is performed. Thresholding is done relative to a common level of reaction progress/relative critical reaction resource availability for all the curves. According to various embodiments, the ensemble of curves are analyzed to come up with a fluorescence threshold which can be used to determine the level of Gene Expression ($C_q$ <-> $C_T$) for each curve.

The $C_T$ is determined as the fractional cycle when the amplification curve's fluorescence level reaches this threshold. As a result, the fluorescence threshold is an ensemble value and special measures have to be taken to handle ensemble outliers. According to some embodiments, in the current algorithm, a measure of RT-PCR reaction progress/relative critical reaction resource availability is used to choose the fluorescence threshold for each curve. The parameterized efficiency curve $e(n\Delta x, X_{shift}, \alpha_{bend}, x_0)$ can be a measure of reaction progress/relative critical reaction resource availability. For simplicity upon writing the efficiency as $e(n)$, then the following expression can be used as a preferred measure of reaction progress/relative critical reaction resource availability can be approximated as:

$$\eta_{reaction\ progress} \approx e\left(n\right).$$

Notice that, for positive $x_0$ (relative shift adjustment parameter), the efficiency is unity at the start of the reaction and decreases to zero as the number of cycles n becomes large. Allowing negative values of $x_0$, causes the efficiency to be less than unity at cycle 0. This can be an indicator of contamination or of non-specific PCR amplification. As a result, the reaction progress/relative critical reaction resource availability can be determined for each curve independent of the other curves in the ensemble of curves being analyzed. By setting the curve thresholds relative to a common level of reaction progress/relative critical reaction resource availability, it becomes easier to possibly compare curves generated from wells on different plates and differences in certain types of conditions.

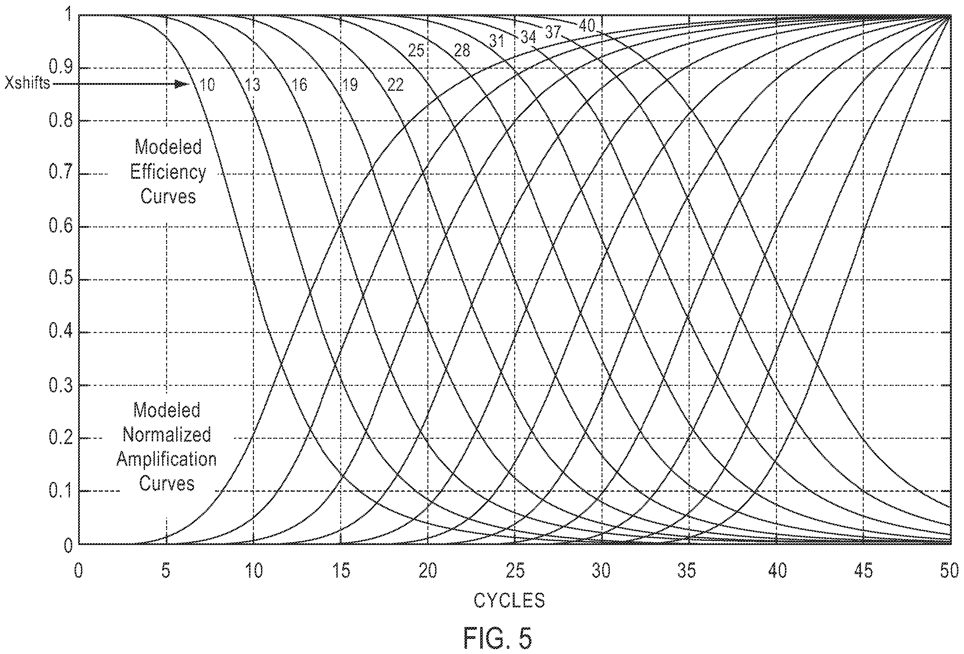

In FIG. 5, a series of graphs demonstrating the impact that an $X_{shift}$ parameter may have on a modeled efficiency and modeled amplification curves is displayed. As one of ordinary skill in the art would understand, the cycle threshold relates to the initial concentration or copy number of a target oligonucleotide in a sample. For various embodiments, FIG. 5 displays how a modeled efficiency curve and a corresponding modeled amplification curve vary as a function of an $X_{shift}$ parameter.

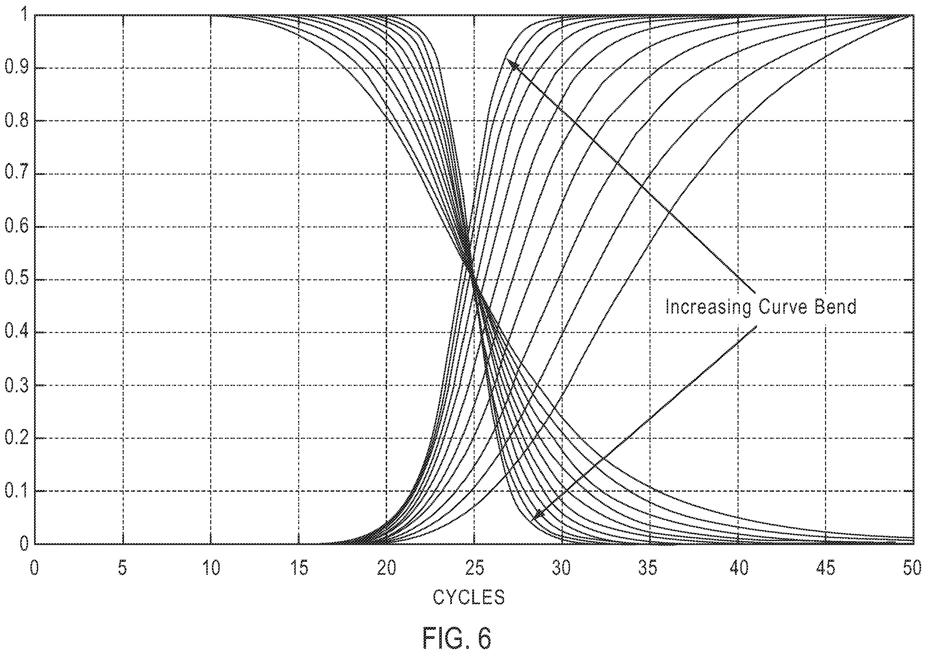

FIG. 6 graphically depicts the impact that an $\alpha_{bend}$ parameter may have on a modeled efficiency and a modeled amplification curve. According to various embodiments of methods and systems, an $\alpha_{bend}$ parameter may vary between about 0.15 to about 2.0. As shown in FIG. 6, $\alpha_{bend}$ is varied between about 0.6 to about 1.0 for complementary sets of a modeled efficiency curve and a modeled amplification curve.

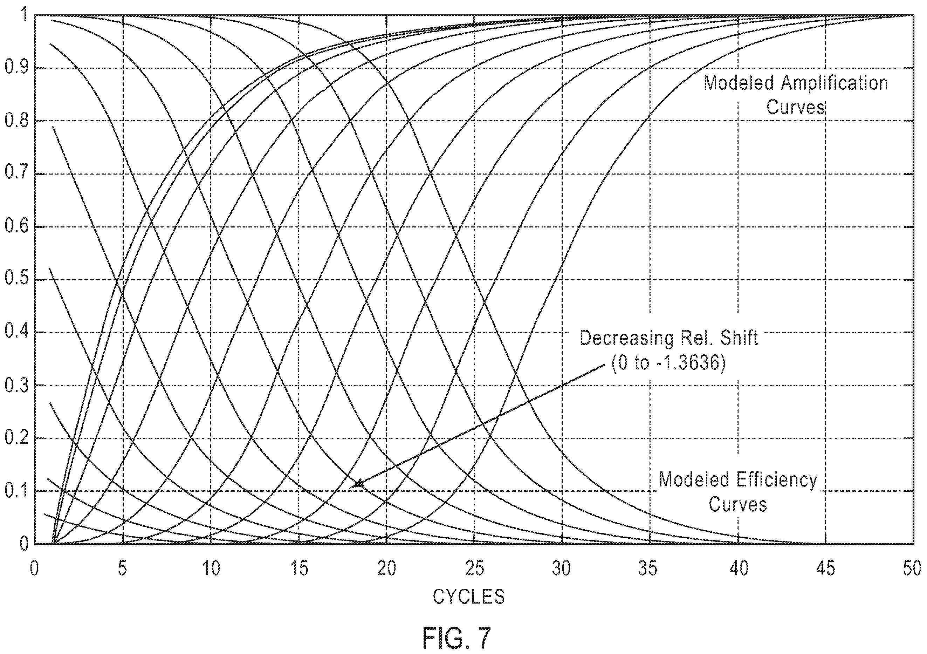

The graphic depiction of the impact that an $X_0'$ relative shift parameter may have on a modeled efficiency and modeled amplification curves is shown in FIG. 7. According to various embodiments, $x_0'$ may be between about -2 to about 0.3. As the $x_0'$ term is related to an initial cycle, $x_0$, as well as $X_{shift}$, it relates to the initial concentration or copy number. FIG. 7 depicts how corresponding sets of modeled efficiency and modeled amplification curves vary as an $x_0'$ relative shift parameter varies from about 0 to about -1.36, according to some embodiments.

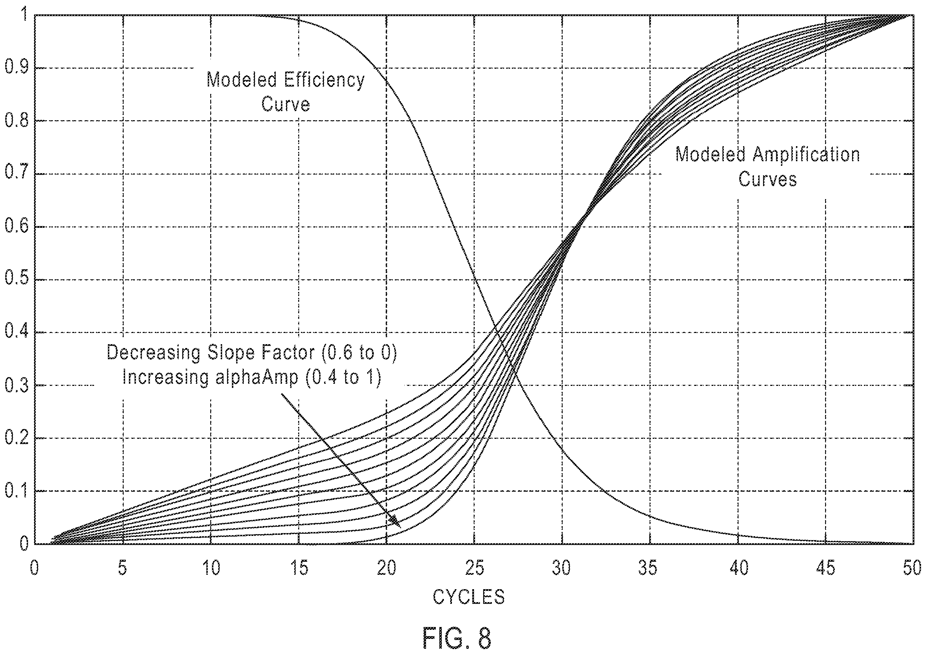

FIG. 8 graphically depicts the impact that $\alpha_{amp}$ and $\Theta_{rising\ baseline}$ parameters may have on a modeled amplification curves. According to various embodiments of methods and systems of the present teachings, the sum of the $\alpha_{amp}$ and $\Theta_{rising}$ baseline parameters is approximately 1. For example, in the limit, for a sample with no amplification, $\alpha_{amp}= 0$, then the rising baseline term would be approximately 1. In FIG. 8, for a decreasing $\Theta_{rising}$ baseline parameter over values from 0.6 to 0 and increasing $\alpha_{amp}$ over values of from 0.4 to 1, a set of modeled amplification curves is shown.

For various embodiments of methods and systems as indicated in FIG. 1, step 30, after a complementary set of a modeled efficiency and a modeled amplification curve has been created for a sample amplification curve, a cycle threshold may be determined. For various embodiments, a cycle threshold from a modeled sample amplification curve may be determined based on a selected value on a corresponding modeled efficiency curve. In various embodiments, a cycle threshold so determined may be referred to as a cycle relative threshold, or $C_{RT}$.

As previously discussed, in reference to FIG. 4, curves I, II, and III correspond to a modeled efficiency curve, an actual sample amplification curve, and a modeled amplification curve, respectively. According to various embodiments for a determination of a $C_{RT}$ value from a modeled efficiency and a corresponding modeled amplification curve, first a reference threshold value on a modeled amplification curve is determined based on a selected efficiency on a modeled efficiency curve. In various embodiments, a selected efficiency of about 0.275 is a value, for example, used to determine a reference threshold value. In the exemplary representation of FIG. 4, point A on curve I corresponds to a point of intersection on the modeled efficiency curve for efficiency = 0.275. The reference threshold level is shown as point B on curve III. After the determination of a reference threshold value, in various embodiments, a relative threshold fractional value may be determined. According to various embodiments, the relative threshold fraction is set using a relative threshold fraction of about 0.67. In FIG. 4, the reference threshold value is multiplied by the relative threshold fraction to determine the relative threshold fraction, as indicated by point C on curve III. According to various embodiments, an unadjusted $C_{RT}$ value is the cycle crossing corresponding to a relative threshold fraction setting, and is indicated in FIG. 4 as point D on the abscissa. For various embodiments, a $C_{RT}$ value determined for a sample amplification curve is the unadjusted $C_{RT}$ value minus a $C_{RT}$ adjustment factor, which factor is about 1.5 cycles. The average $C_t$ from AutoCt ID wells measurements on a large number of "identical" biological samples. The average $C_{RT}$ measurement is obtained for the same database of "identical" biological samples. This adjustment factor is obtained as the difference between this average $C_{RT}$ value and the average CTvalue. In FIG. 4, a determined sample $C_{RT}$ is indicated as point E on the abscissa.

Various embodiments of values for a selected efficiency, a relative threshold fraction, and a $C_{RT}$ adjustment factor may be determined empirically based on a database of sample curves analyzed. In various embodiments of methods and systems, values for a selected efficiency may be in a range up to about 0.5. A good starting value for the efficiency threshold would be a value less than 0.5 when the PCR reaction starts to slow down appreciably. A database of ID wells measurements on a large number of "identical" biological samples is needed to determine the best combination of efficiency threshold and relative amplification fraction. As one skilled in the art would understand, a manual hand optimization can be used to select combinations of efficiency threshold and relative amplification fraction values that result in low measurement standard deviation. In general, this process can be subject to a 2 parameter constrained optimization process where the objective function is the standard deviation. This constrained optimization process can be used to select the best pair of efficiency threshold and relative amplification fraction parameters. For various embodiments, a selected efficiency may be selected from between about 0.15 to 0.5. In various embodiments, a relative threshold fraction may be between about 0.1 to about 1.25. According to various embodiments, a $C_{RT}$ adjustment factor may vary from between about -2 to about 2.

In various embodiments, an additional bias adjustment may improve linearity of the modeled amplification curves. The ability to create parameterized modeled amplification curves can be used to create a detailed database of modeled amplification curves as a function of the gene expression proxy $X_{shift} + X_0$. In one embodiment, a database of modeled amplification curves can be created for values $X_{shift} + X_0$ of between 0.5 and 50 in increments of 0.1. Here, $X_{shift} + X_0$ is being used as a proxy for $C_q$. The values of the parameters used to create the modeled curves can be randomized slightly and noise can be added to each curve. At each nominal parameter level a curve can be generated up to 64 times. This results in a very large database of curves for which the nominal values of the $C_q$ proxy is known. Upon getting the $C_{RT}$ values for the curves in the database, the $C_{RT}$ "bias" regarding the known $C_q$ proxy values can be calculated. As a result, a bias table for $C_{RT}$ vs the $C_{RT}$ "bias". This makes it possible to bias adjust $C_{RT}$ values so that it is more linear in the range of 0.5-50 $C_q$ cycles. Notice that because the $C_{RT}$ algorithm is based on modeled curves and constrained optimization, it is possible to make projected $C_q$ measurements beyond the maximum number of PCR amplification cycles used (typically 40 cycles). For example, the amplification curve may be projected out to 40-50 cycles. In various embodiments, a bias adjustment may be between +/-0.00 -1.5$C_q$. but would be typically less than 0.5 $C_q$.

One skilled in the art would recognize that the $C_{RT}$ algorithm, according to various embodiments, produces several parameters that could be used as a basis for a $C_q$ measurement (after appropriately bias shifting to align the results with the average values of other $C_q$ measurements). The fractional cycle value associated with the reference threshold on the amplified curve produced from the reference efficiency threshold on the efficiency curve (the Reference Cycle) is a good alternative basis for a $C_q$. This is equivalent to setting the relative threshold fraction to 1.0 in the preferred version of $C_{RT}$ algorithm and produces very good results. Alternatively, the previously described $C_q$ "proxy", $X_{shift} + x_0$, could also be used as a basis for a $C_q$ measurement. One skilled in the art would also recognize that the best-fit efficiency curve could also be used to produce efficiency corrected versions of $C_{RT}$, the Reference Cycle, or $X_{shift} + x_0$ and using those corrected versions as the basis for the $C_q$ measurement. An efficiency correction would be for instance to find the equivalent cycle if the efficiency was constant at 1.0 (or another value) rather than variable.

Regarding FIG. 1, step 40, as previously discussed, and as one of ordinary skill in the art may readily recognize, there are various ways of outputting cycle threshold information; for example, but not limited by efficiency and amplification curve data, final cycle threshold values, and cycle threshold quality scores, to an end user in numerous formats using numerous devices. For example, with respect to format of efficiency and amplification curve data, the data may be presented in a graphical format, as a written report, or combinations thereof. With respect to output devices, cycle threshold information may be output to devices such as, but not limited by a printer, a cathode ray tube (CRT) display, a liquid crystal display (LCD), and a light-emitting diode (LED) display.

According to various embodiments of methods and systems for the analysis of PCR amplification curve data of the present teachings, a plurality of tests may be done on sample data to identify a non-amplified sample. For various embodiments, characteristics of a sample amplification curve, a constrained parameter inversion of each sample amplification curve, and an ensemble of curves being analyzed may be used to assess whether a sample is a non-amplified sample. In various embodiments, characteristics, such as, but not limited by a normalized modeled curve amplitude, a weighted RMS (Root Mean Square) curve model fitting error, and an individual sample amplification curve RMS noise level, may help to determine whether the curve is from an amplified well or is from a non-amplified, and possibly contaminated, well.

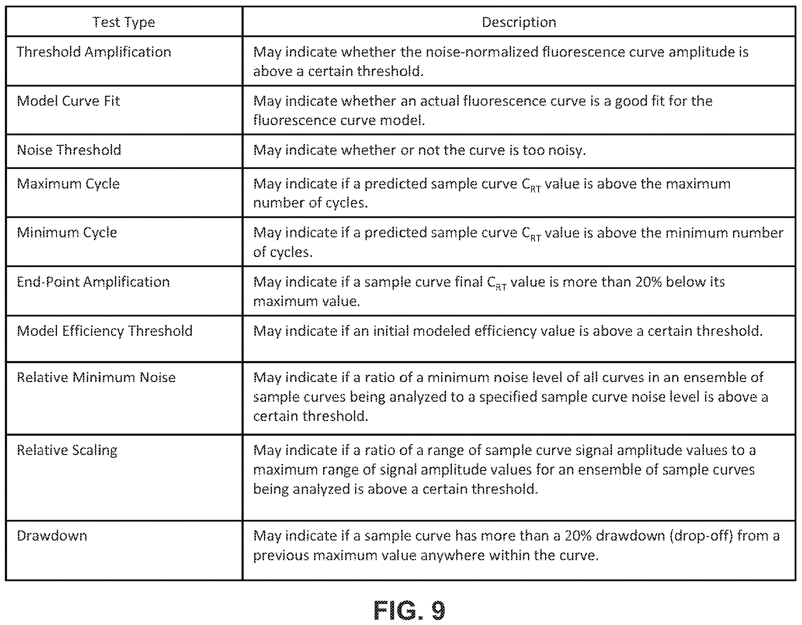

In FIG. 9, a variety of tests are listed, which may be used to evaluate whether or not a sample may be non-amplified. The tests may be implemented by a processor 304 as depicted in FIG. 3. According to various embodiments, sample amplification data may have to pass all of the tests to be classified as amplified. In other embodiments, at least one test may be used to evaluate whether sample amplification data may be classified as amplified or non-amplified.

A threshold amplification test may indicate whether the noise-normalized amplification curve amplitude is above a certain threshold. If the amplification curve is above the threshold, this may indicate amplification. The threshold is higher for noisier curves and smaller $C_{RT}$ values.

A model curve fit test may indicate whether the actual amplification curve is a good fit for the best-fit parameter fluorescence curve model. In some embodiments, the model curve fit test may have an RMS value of 0.075. If the actual amplification curve fits the modeled amplification curve well, this may indicate amplification.

A noise threshold test may indicate whether the curve is too noisy. In some embodiments, the threshold used may be 0.0625. If the amplification curve is above the noise threshold, it is noisy and may indicate non-amplification.

A maximum cycle test may indicate whether the predicted $C_{RT}$ value is above the maximum number of cycles, indicating amplification. In an embodiment, the threshold for this test will be extended (by about 8-10 cycles) beyond the maximum number of cycles. This would allow the algorithm to predict $C_{RT}$ values beyond the maximum number of cycles.

A minimum cycle test may indicate whether the predicted $C_{RT}$ value is below a minimum number of cycles, indicating amplification. In some embodiments, the minimum number of cycles may be 1.25.

An end-point amplification test may indicate whether the final value of the curve is more than a certain threshold amount below its maximum value. If the curve is less than the threshold amount below the maximum value, this may indicate amplification. In some embodiments, the threshold may be set at about 20% below its maximum value.

A model efficiency threshold test may indicate whether the initial modeled efficiency value is above a certain threshold, indicating amplification. In some embodiments, the threshold may be set at 0.65. The initial efficiency value is almost always 1. However, in certain cases, e.g. when the well is contaminated, this initial efficiency can fall below unity. As such, this test may also indicate whether a sample or well has been contaminated or it may be an indicator of non-specific PCR amplification.

A relative noise test indicates whether the ratio of the minimum noise level of all the curves in the ensemble being analyzed together to the current curve's noise level is above a certain threshold. In some embodiments, the threshold may be 0.015. If the curve has a ratio above this threshold, it may indicate amplification. A noisy curve may indicate interference from other sample wells.

A relative scaling test may indicate whether the ratio of the range of the fluorescence values of the curves to the maximum range of fluorescence values for all the curves in the analysis ensemble is above a certain threshold. In some embodiments, the efficiency threshold is 0.275. A ratio above the threshold may indicate amplification.

A drawdown test may indicate whether the fluorescence curve has more than a certain drop-off, or drawdown, from a previous maximum value anywhere within the curve. If the amplification curve is more than the threshold drop-off, this may indicate non-amplification. In some embodiments, the drop-off is 20% from a previous maximum value.

A straight line test may indicate a threshold to determine whether the amplification curve is best characterized by a straight line. In some embodiments, if the $R^2$ for the straight model is above 0.99, the amplification curve is considered to be associated with a non-amplified well. Similarly, this straight line model can be used to count the number of times the amplification curve crosses the line. If the number of crosses is too large, the amplification curve may be considered to be associated with a non-amplified well.

In that regard, some of the tests may be stronger indicators of whether or not sample data has been amplified. According to various embodiments, a threshold amplification test may be utilized as a strong indicator of whether or not sample data has been amplified. In various embodiments, a threshold amplification test may indicate whether a noise-normalized amplification curve amplitude is above a certain threshold. Conceptually, as one of ordinary skill in the art is apprised, noise is known to impact a determination of a threshold value, as thresholds are typically defined as a signal exceeding a defined noise level. For various embodiments of a threshold amplification test, a noise-normalized amplification factor derived from a ratio of $\alpha_{amp}$ to an expression for noise is a may be used. According to various embodiments, a threshold level a may be set for a noise-normalized amplification factor so determined, which threshold may set the limit determining whether or not sample data has been amplified.

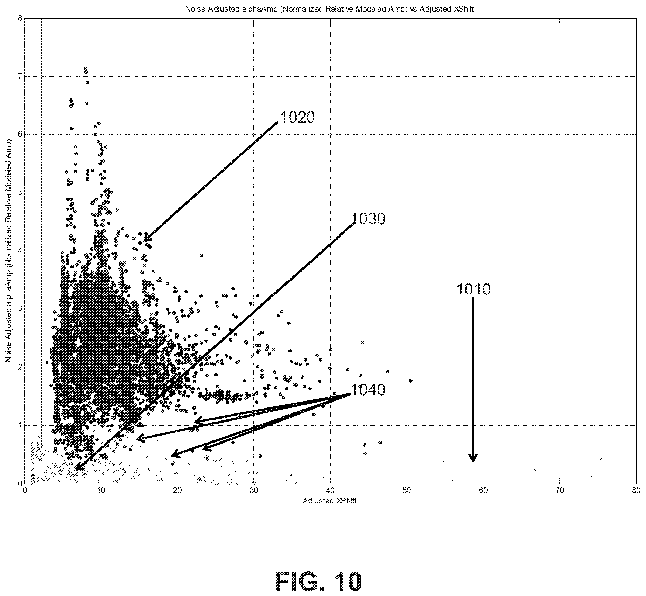

For example, with reference to FIG. 10, it was determined empirically that, for each amplification curve, the threshold value should be set higher when it is noisier, has smaller $C_{RT}$ values, and/or has smaller $\alpha_{bend}$ values. In the preferred embodiment, rather than making the threshold higher for noisier amplification curves with smaller $C_{RT}$ and/or $\alpha_{bend}$ values, the noise-normalized amplification curve amplitude is scaled to become smaller than the original amplification curve amplitude without changing the threshold values. Thus we could define values of a threshold curve as a function of a curve's adjusted $X_{shift}$ value. The threshold test is true when the amplification curve's noise-normalized amplitude is above the threshold curve. The threshold value is determined based on the curve's adjusted $X_{shift}$ value. A false value for this test is an indication that the curve/well is non-amplified.

FIG. 10 shows plots of noise-normalized amplification curve amplitudes versus adjusted $X_{shift}$ values for various amplified and non-amplified curves/wells. The threshold curve as a function of the adjusted $X_{shift}$ is clearly indicated and defined for this embodiment by threshold curve 1010 in FIG. 10. It should be noted that curves/wells with adjusted $X_{shift}$ values that are smaller than a threshold value are called nonamplified. In some embodiments, the threshold value for the adjusted $X_{shift}$ is 2.250.

In the FIG. 10, amplification curves 1020 and their associated wells that are determined to be "amplified." Amplification curves 1030 and their associated wells are determined to be "non-amplified." Amplification curves 1040 and their associated wells that were annotated by a human to have been incorrectly determined to be amplified or non-amplified.

The dataset in this example represents over 7700 wells. In this dataset there were 8 missed calls. One of the missed calls was a false positive (FP) meaning that the curve/well was called amplified but was annotated as non-amplified. The other 7 missed calls was a false negative (FN) meaning that the curve/well was called non-amplified but was annotated as amplified. A few of those FN calls were called non-amplified because the $C_{RT}$ value was greater than the maximum number of cycles. Otherwise, in the cases, the algorithm would have correctly called the corresponding amplification curves amplified.

In another example, according to various embodiments, an expression for noise may be derived by the square root of the ratio of an amplification curve's approximate RMS noise to a reference noise value. For various embodiments, a noise-normalized amplification factor may be derived from a ratio of $\alpha_{amp}$ for a sample amplification curve to an expression of noise so determined. According to various embodiments of a threshold amplification test, then for noisier amplification curves, with approximate RMS noise values above a reference noise value, the noise-normalized amplification curve amplitude becomes less than an original amplification curve amplitude. In various embodiments, such a result may make a amplification curve less likely to be called amplified. Conversely, for various embodiments, for less noisy amplification curves, with approximate RMS noise values below a reference noise value, a noise-normalized amplification curve amplitude becomes greater than an original amplification curve amplitude. According to various embodiments, such as result may make a curve more likely to be called amplified.

For various embodiments of methods and systems for the analysis of PCR amplification curve data, a quality value may be determined for every cycle threshold value determined for each sample. According to various embodiments, a probability that a cycle threshold determined for each sample is a correct cycle threshold may be made. As such, a quality value may be used as additional information for an end user to evaluate a determined cycle threshold for a sample.

**Claims**

1. A computer-implemented method (100) for determining a cycle threshold for a PCR amplification curve, the method comprising:

   receiving (10) a data set for a plurality of biological samples for a PCR amplification reaction, wherein the data set includes a plurality of amplification curves, each amplification curve associated with a biological sample of the plurality of biological samples;

   performing (20) a constrained nonlinear optimization comprising a fit of each of the plurality of amplification curves to a modeled amplification curve to determine a best-fit set of parameters for a modeled efficiency curve and the modeled amplification curve, and wherein the best-fit set of parameters are used to create the modeled efficiency curve and the modeled amplification curve for each of the plurality of amplification curves; and

   determining (30) a relative cycle threshold value, $C_{RT}$, for each biological sample by using the modeled efficiency curve and the modeled amplification curve,

   wherein the constrained nonlinear optimization is a constrained non-linear parameter optimization,

   wherein the best-fit set of parameters for the modeled efficiency curve and the modeled amplification curve comprises three parameters being a curve shift parameter, a curve bend parameter, and a curve shift adjustment parameter, and comprises additionally two parameters being an amplification curve rising baseline slope parameter and an amplification curve relative amplification parameter for the modeled amplification curve, and said modeled efficiency curve is of a 3-parameter efficiency model using said three parameters and said modeled amplification curve is of a 5-parameter amplification model using said three parameters and said additionally two parameters,

   wherein using the modeled efficiency curve and the modeled amplification curve to determine the $C_{RT}$ value for each biological sample comprises:

   - using a selected efficiency value on the respective modeled efficiency curve to determine a reference threshold value on the respective modeled amplification curve;
   - multiplying the reference threshold value by a relative threshold fraction to determine a relative threshold fraction value on the respective modeled amplification curve to identify an unadjusted $C_{RT}$; and
   - using a cycle adjustment factor to adjust the unadjusted $C_{RT}$ to obtain the $C_{RT}$,

   wherein determining the $C_{RT}$ is based on the efficiency value being selected from between about 0.15 to 0.5, and wherein the relative threshold fraction value is between about 0.1 and 1.25, and wherein the cycle adjustment factor is between about -2 and 2.

2. The computer-implemented method (100) of claim 1, further comprising identifying non-amplified samples, wherein the identifying non-amplified samples comprises performing a test selected from the group consisting of:

   - threshold amplification indicating whether a noise-normalized fluorescence curve amplitude is above a certain first threshold,
   - model curve fit indicating whether an actual fluorescence curve fits a fluorescence curve model,
   - noise threshold,
   - maximum cycle indicating if a predicted sample curve $C_{RT}$ value is above a maximum number of cycles,
   - minimum cycle indicating if a predicted sample curve $C_{RT}$ value is above a minimum number of cycles,
   - end-point amplification indicating if a sample curve final $C_{RT}$ value is more than 20% below the maximum value,
   - model efficiency threshold indicating if an initial modeled efficiency value is above a certain second threshold,
   - relative minimum noise indicating if a ratio of minimum noise level of all curves in an ensemble of sample curves being analyzed to a specific sample curve noise level is above a certain third threshold,
   - relative scaling indicating if a ratio of a range of sample curve signal amplitude values to a maximum range of signal amplitude values for an ensemble of sample curves being analyzed is above a certain fourth threshold, or
   - drop-off indicating if a sample curve has more than a 20% drop-off from a previous maximum value anywhere within the sample curve.

3. The computer-implemented method (100) of claim 1, further comprising generating a projection of an estimated fractional qPCR cycle value, $C_q$, beyond a maximum number of PCR cycles.

4. A computer-readable medium encoded with instructions, executable by a processor, for determining a cycle threshold for a PCR amplification curve, the instructions comprising instructions for performing a method of one of claims 1 - 3.

5. A system (300) for determining a cycle threshold for a PCR amplification curve, the system comprising:

a processor (304); and
a memory (310) storing instructions executable by the processor (304), the instructions comprising instructions for performing a method of one of claims 1 - 3.

**Patentansprüche**

1. Computerimplementiertes Verfahren (100) zum Bestimmen eines Zyklusschwellenwerts für eine PCR-Amplifikationskurve, wobei das Verfahren Folgendes umfasst:

Empfangen (10) eines Datensatzes für mehrere biologische Proben für eine PCR-Amplifikationsreaktion, wobei der Datensatz mehrere Amplifikationskurven beinhaltet, wobei jede Amplifikationskurve mit einer biologischen Probe der mehreren biologischen Proben assoziiert ist;
Durchführen (20) einer eingeschränkten nichtlinearen Optimierung, die eine Übereinstimmung jeder der mehreren Amplifikationskurven mit einer modellierten Amplifikationskurve umfasst, um einen Parametersatz für eine bestmögliche Übereinstimmung für eine modellierte Effizienzkurve und die modellierte Amplifikationskurve zu bestimmen, und wobei der Parametersatz für eine bestmögliche Übereinstimmung verwendet wird, um die modellierte Effizienzkurve und die modellierte Amplifikationskurve für jede der mehreren Amplifikationskurven zu erstellen; und
Bestimmen (30) eines relativen Zyklusschwellenwerts ($C_{RT}$) für jede biologische Probe durch Verwenden der modellierten Effizienzkurve und der modellierten Amplifikationskurve, wobei die eingeschränkte nichtlineare Optimierung eine eingeschränkte nichtlineare Parameteroptimierung ist, wobei der Parametersatz für eine bestmögliche Übereinstimmung für die modellierte Effizienzkurve und die modellierte Amplifikationskurve drei Parameter umfasst, die ein Kurvenverschiebungsparameter, ein Kurvenkrümmungsparameter und ein Kurvenverschiebungseinstellungsparameter sind, und zusätzlich zwei Parameter umfasst, die ein Parameter für die ansteigende Basislinienneigung der Amplifikationskurve und ein Parameter für die relative Amplifikation der Amplifikationskurve für die modellierte Amplifikationskurve sind, und wobei die modellierte Effizienzkurve einem 3-Parameter-Effizienzmodell unter Verwendung der drei Parameter entspricht und die modellierte Amplifikationskurve einem 5-Parameter-Amplifikationsmodell unter Verwendung der drei Parameter und der zusätzlichen zwei Parameter entspricht,
wobei das Verwenden der modellierten Effizienzkurve und der modellierten Amplifikationskurve, um den $C_{RT}$-Wert für jede biologische Probe zu bestimmen, Folgendes umfasst:

- Verwenden eines ausgewählten Effizienzwerts auf der jeweiligen modellierten Effizienzkurve, um einen Referenzschwellenwert auf der jeweiligen modellierten Amplifikationskurve zu bestimmen;
- Multiplizieren des Referenzschwellenwerts mit einem relativen Schwellenwertanteil, um einen relativen Schwellenwertanteilswert auf der jeweiligen modellierten Amplifikationskurve zu bestimmen, um einen nicht eingestellten $C_{RT}$ zu identifizieren; und
- Verwenden eines Zykluseinstellungsfaktors, um den nicht eingestellten $C_{RT}$ einzustellen, um den $C_{RT}$ zu erhalten,

wobei das Bestimmen des $C_{RT}$ auf dem Effizienzwert basiert, der aus einem Bereich zwischen etwa 0,15 und 0,5 ausgewählt wird, und wobei der relative Schwellenwertanteilswert zwischen etwa 0,1 und 1,25 liegt, und wobei der Zykluseinstellungsfaktor zwischen etwa -2 und 2 liegt.

2. Computerimplementiertes Verfahren (100) nach Anspruch 1, das ferner das Identifizieren von nicht-amplifizierten Proben umfasst, wobei das Identifizieren von nicht-amplifizierten Proben das Durchführen eines Tests umfasst, der aus der Gruppe ausgewählt ist, die aus Folgendem besteht:

- Schwellenwertamplifikation, die angibt, ob eine rauschnormierte Fluoreszenzkurvenamplitude über einem gewissen ersten Schwellenwert liegt,

- Modellkurvenübereinstimmung, die angibt, ob eine tatsächliche Fluoreszenzkurve mit einem Fluoreszenzkurvenmodell übereinstimmt,
- Rauschschwellenwert,
- maximalem Zyklus, der angibt, ob ein vorhergesagter Probenkurven-$C_{RT}$-Wert über einer maximalen Anzahl von Zyklen liegt,
- minimalem Zyklus, der angibt, ob ein vorhergesagter Probenkurven-$C_{RT}$-Wert über einer minimalen Anzahl von Zyklen liegt,
- Endpunktamplifikation, die angibt, ob der Probenkurven-$C_{RT}$-Endwert mehr als 20 % unter dem maximalen Wert liegt,
- Modelleffizienzschwellenwert, der angibt, ob ein anfänglicher modellierter Effizienzwert über einem gewissen zweiten Schwellenwert liegt,
- relativem minimalen Rauschen, das angibt, ob ein Verhältnis des minimalen Rauschpegels aller Kurven in einer Gruppe von analysierten Probenkurven zu einem spezifischen Probenkurvenrauschpegel über einem gewissen dritten Schwellenwert liegt,
- relativer Skalierung, die angibt, ob ein Verhältnis eines Bereichs von Probenkurvensignalamplitudenwerten zu einem maximalen Bereich von Signalamplitudenwerten für eine Gruppe von analysierten Probenkurven über einem gewissen vierten Schwellenwert liegt, oder
- Abnahme, die angibt, ob eine Probenkurve mehr als 20 % Abnahme von einem vorherigen maximalen Wert an einer beliebigen Stelle innerhalb der Probenkurve aufweist.

3. Computerimplementiertes Verfahren (100) nach Anspruch 1, das ferner ein Erzeugen einer Projektion eines geschätzten fraktionierten qPCR-Zykluswerts ($C_q$) über eine maximale Anzahl von PCR-Zyklen hinaus umfasst.

4. Computerlesbares Medium, das mit Anweisungen codiert ist, die durch einen Prozessor ausführbar sind, zum Bestimmen eines Zyklusschwellenwerts für eine PCR-Amplifikationskurve, wobei die Anweisungen Anweisungen zum Durchführen eines Verfahrens nach einem der Ansprüche 1-3 umfassen.

5. System (300) zum Bestimmen eines Zyklusschwellenwerts für eine PCR-Amplifikationskurve, wobei das System Folgendes umfasst:

einen Prozessor (304); und
einen Speicher (310), der Anweisungen speichert, die durch den Prozessor (304) ausführbar sind, wobei die Anweisungen Anweisungen zum Durchführen eines Verfahrens nach einem der Ansprüche 1-3 umfassen.

## Revendications

1. Procédé mis en œuvre par ordinateur (100) destiné à déterminer un seuil de cycle pour une courbe d'amplification PCR, le procédé comprenant :

la réception (10) d'un ensemble de données pour une pluralité d'échantillons biologiques pour une réaction d'amplification PCR, l'ensemble de données comportant une pluralité de courbes d'amplification, chaque courbe d'amplification étant associée à un échantillon biologique de la pluralité d'échantillons biologiques ;
la réalisation (20) d'une optimisation non linéaire contrainte comprenant un ajustement de chacune de la pluralité de courbes d'amplification à une courbe d'amplification modélisée pour déterminer un ensemble de paramètres le mieux adapté pour une courbe d'efficacité modélisée et la courbe d'amplification modélisée, et l'ensemble de paramètres le mieux adapté utilisé pour créer la courbe d'efficacité modélisée et la courbe d'amplification modélisée pour chacune de la pluralité de courbes d'amplification ; et
la détermination (30) d'une valeur seuil de cycle relatif, $C_{RT}$, pour chaque échantillon biologique à l'aide de la courbe d'efficacité modélisée et la courbe d'amplification modélisée,
l'optimisation non linéaire contrainte étant une optimisation de paramètre non linéaire contrainte,
l'ensemble de paramètres le mieux adapté pour la courbe d'efficacité modélisée et la courbe d'amplification modélisée comprenant trois paramètres étant un paramètre de décalage de courbe, un paramètre de courbure de courbe et un paramètre d'ajustement de décalage de courbe, et comprenant en outre deux paramètres étant un paramètre de pente de base montante de courbe d'amplification et un paramètre d'amplification relative à la courbe d'amplification pour la courbe d'amplification modélisée, et
ladite courbe d'efficacité modélisée est d'un modèle d'efficacité à 3 paramètres utilisant lesdits trois paramètres et ladite courbe d'amplification modélisée est d'un modèle d'amplification à 5 paramètres utilisant lesdits trois paramètres et lesdits deux autres paramètres, l'utilisation de la courbe d'efficacité modélisée et de la courbe d'amplification modélisée pour déterminer la valeur $C_{RT}$ pour chaque échantillon biologique comprenant :

- l'utilisation d'une valeur d'efficacité choisie sur la courbe d'efficacité modélisée respective pour déterminer une valeur seuil de référence sur la courbe d'amplification modélisée respective ;
- la multiplication de la valeur seuil de référence par une fraction de seuil relative pour déterminer une valeur de fraction seuil relative sur la courbe d'amplification modélisée respective afin d'identifier un $C_{RT}$ non ajusté ; et
- l'utilisation d'un facteur d'ajustement de cycle pour ajuster le $C_{RT}$ non ajusté afin d'obtenir le $C_{RT}$,

la détermination du $C_{RT}$ étant basée sur la valeur d'efficacité choisie entre environ 0,15 et 0,5, et la valeur de fraction seuil étant comprise entre environ 0,1 et 1,25, et die facteur d'ajustement de cycle étant compris entre environ -2 et 2.

2. Procédé mis en œuvre par ordinateur (100) selon la revendication 1, comprenant en outre l'identification d'échantillons non amplifiés, l'identification des échantillons non amplifiés comprenant la réalisation d'un test choisi dans le groupe constitué par :

- l'amplification seuil indiquant si une amplitude de courbe de fluorescence normalisée au bruit est supérieure à un certain premier seuil,
- l'ajustement de la courbe du modèle indiquant si une courbe de fluorescence réelle correspond à un modèle de courbe de fluorescence,
- le seuil de bruit,
- le cycle maximal indiquant si une valeur $C_{RT}$ de la courbe d'échantillon prédite est supérieure à un nombre maximal de cycles,
- le cycle minimal indiquant si une valeur de $C_{RT}$ de la courbe d'échantillon prédite est supérieure à un nombre minimal de cycles,
- l'amplification du point d'extrémité indiquant si la valeur finale de $C_{RT}$ de la courbe d'échantillon est inférieure de plus de 20 % à la valeur maximale,
- le seuil d'efficacité de modèle indiquant si une valeur d'efficacité modélisée initiale est supérieure à un certain second seuil,
- le bruit minimal relatif indiquant si un rapport du niveau de bruit minimal de toutes les courbes d'un ensemble de courbes d'échantillon analysées à un niveau de bruit de courbe d'échantillon spécifique est supérieur à un certain seuil,
- l'échelle relative indiquant si un rapport d'une plage de valeurs d'amplitude de signal de courbe d'échantillon à une plage maximale de valeurs d'amplitude de signal pour un ensemble de courbes d'échantillon analysées est supérieur à un certain quatrième seuil, ou
- la baisse indiquant si une courbe d'échantillon présente une baisse de plus de 20 % par rapport à une valeur maximale précédente n'importe où dans la courbe d'échantillon.

3. Procédé mis en œuvre par ordinateur (100) selon la revendication 1, comprenant en outre la génération d'une projection d'une valeur de cycle qPCR fractionnaire estimée, $C_q$, au-delà d'un nombre maximal de cycles PCR.

4. Support lisible par ordinateur codé par des instructions, exécutables par un processeur, pour déterminer un seuil de cycle pour une courbe d'amplification PCR, les instructions comprenant des instructions pour effectuer un procédé selon l'une des revendications 1 à 3.

5. Système (300) de détermination d'un seuil de cycle pour une courbe d'amplification PCR, le système comprenant :

un processeur (304) ; et
une mémoire (310) stockant des instructions exécutables par le processeur (304), les instructions comprenant des instructions pour exécuter un procédé selon l'une des revendications 1 à 3.

100
↓

RECEIVING A DATA SET FOR A PLURALITY OF BIOLOGICAL SAMPLES FOR A PCR AMPLIFICATION REACTION

10

PERFORMING A CONSTRAINED NONLINEAR OPTIMIZATION COMPRISING A FIT OF EACH AMPLIFICATION CURVE TO A COMPLEMENTARY MODELED AMPLIFICATION CURVE TO DETERMINE A BEST-FIT SET OF PARAMETERS FOR EACH MODELED EFFICIENCY CURVE AND ASSOCIATED AMPLIFICATION CURVE

20

DETERMINING A CYCLE THRESHOLD VALUE FOR EACH BIOLOGICAL SAMPLE BASED ON A COMPLEMENTARY RELATIONSHIP OF THE MODELED EFFICIENCY CURVE TO THE MODELED AMPLIFICATION CURVE.

30

OUTPUT RESULTS

40

FIG. 1

FIG. 2

**FIG. 3**

FIG. 4

FIG. 5

EP 2 558 971 B1

FIG. 6

FIG. 7

EP 2 558 971 B1

Modeled Efficiency
Curve

Modeled Amplification
Curves

Decreasing Slope Factor (0.6 to 0)
Increasing alphaAmp (0.4 to 1)

CYCLES

FIG. 8

| Test Type | Description |
| --- | --- |
| Threshold Amplification | May indicate whether the noise-normalized fluorescence curve amplitude is above a certain threshold. |
| Model Curve Fit | May indicate whether an actual fluorescence curve is a good fit for the fluorescence curve model. |
| Noise Threshold | May indicate whether or not the curve is too noisy. |
| Maximum Cycle | May indicate if a predicted sample curve $C_{RT}$ value is above the maximum number of cycles. |
| Minimum Cycle | May indicate if a predicted sample curve $C_{RT}$ value is above the minimum number of cycles. |
| End-Point Amplification | May indicate if a sample curve final $C_{RT}$ value is more than 20% below its maximum value. |
| Model Efficiency Threshold | May indicate if an initial modeled efficiency value is above a certain threshold. |
| Relative Minimum Noise | May indicate if a ratio of a minimum noise level of all curves in an ensemble of sample curves being analyzed to a specified sample curve noise level is above a certain threshold. |
| Relative Scaling | May indicate if a ratio of a range of sample curve signal amplitude values to a maximum range of signal amplitude values for an ensemble of sample curves being analyzed is above a certain threshold. |
| Drawdown | May indicate if a sample curve has more than a 20% drawdown (drop-off) from a previous maximum value anywhere within the curve. |

**FIG. 9**

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007260440 A1
- US 5538848 A
- US 6103476 A
- US 5925517 A
- WO 9921881 A
- US 6355421 B
- US 6593091 B
- US 6150097 A
- US 6548250 B
- US 6589743 B
- US 6590091 B
- US 6589250 B
- US 6383752 B
- US 6596490 B
- US 6485901 B

### Non-patent literature cited in the description

- **RUTLEDGE ROBERT G et al.** A kinetic-based sigmoidal model for the polymerase chain reaction and its application to high-capacity absolute quantitative real-time PCR. BMC BIOTECHNOLOGY, BIOMED CENTRAL LTD, vol. 8, 47
- **FURTHER, C. RITZ et al.** qpcR: an R package for sigmoidal model selection in quantitative real-time polymerase chain reaction analysis. *BIOINFORMATICS, (20080701),* vol. 24 (13), 1549-1551
- **BUSTIN SA ; BENES V ; GARSON JA ; HELLEMANS J ; HUGGETT J ; KUBISTA M ; MUELLER R ; NOLAN T ; PFAFFL MW ; SHIPLEY GL.** The MIQE guidelines: minimum information for publication of quantitative real-time PCR experiments. *Clin Chem,* 2009, vol. 55, 611-622
- **TYAGI ; KRAMER.** *Nature Biotechnology,* 1996, vol. 14, 303-308
- **KUBISTA et al.** *SPIE,* 2001, vol. 4264, 53-58
- **SOLINAS et al.** *Nucleic Acids Research,* 2001, vol. 29, E96
- **MHLANGA et al.** *Methods,* 2001, vol. 25, 463-471
- **WHITCOMBE et al.** *Nature Biotechnology,* 1999, vol. 17, 804-807
- **ISACSSON et al.** *Molecular Cell Probes,* 2000, vol. 14, 321-328
- **SVANVIK et al.** *Anal Biochem.,* 2000, vol. 281, 26-35
- **WOLFFS et al.** *Biotechniques,* 2001, vol. 766, 769-771
- **TSOURKAS et al.** *Nucleic Acids Research,* 2002, vol. 30, 4208-4215
- **RICCELLI et al.** *Nucleic Acids Research,* 2002, vol. 30, 4088-4093
- **ZHANG et al.** *Shanghai,* 2002, vol. 34, 329-332
- **MAXWELL et al.** *J. Am. Chem. Soc.,* vol. 124, 9606-9612
- **BROUDE et al.** *Trends Biotechnol.,* 2002, vol. 20, 249-56
- **HUANG et al.** *Chem Res. Toxicol.,* 2002, vol. 15, 118-126
- **YU et al.** *J. Am. Chem. Soc,* 2001, vol. 14, 11155-11161